# EUROPEAN PATENT SPECIFICATION

(11) **EP 0 685 231 B1**
(45) Date of publication and mention of the grant of the patent: **07.07.1999**
(21) Application number: 95105628.2
(22) Date of filing: 29.01.1992
(51) Int. Cl.: A61K 31/53, A61K 33/06, A61K 9/20

(54) **Water-dispersible tablet containing lamotrigine**
Wasserdispergierbare Tablette enthaltend Lamotrigin
Comprimé dispersible dans l'eau à base de lamotrigine

(30) Priority: 22.11.1991 GB 9124807
(43) Date of publication of application: 06.12.1995
(62) Divisional of application: 92903508.7
(73) Proprietor: THE WELLCOME FOUNDATION LIMITED, London NW1 2BP (GB)
(72) Inventor: Fielden, Krystyna Elzbieta, Cheshunt, Hertfordshire, EN8 9NB (GB)
(74) Representative: Thornley, Rachel Mary

(56) References cited:
- EP-A- 0 021 121
- EP-A- 0 247 892
- EP-A- 0 350 701
- EP-A- 0 391 851
- DE-A- 2 016 622
- DATABASE WPI Week 6800, Derwent Publications Ltd., London, GB; AN 67-04040H (00) & JP-B-43 024 078 (NISSAN CHEM. IND. LTD.)

## Description

The present invention relates to a water-dispersible tablet formulation containing lamotrigine (EP Nos. 021,121 and EP 247,892). A patent relating to a water-dispersible tablet containing acyclovir has been granted as EP patent no. 0522128.

Therapeutically active compounds or drugs are frequently administered to patients in tablet form where the drug is intended for oral administration since tablets are an especially convenient pharmaceutical form for manufacture, storage and generally usage. However, problems, may arise with the administration of such tablets to patients who have difficulty in swallowing the tablets (for example, children or more seriously ill patients) especially if the tablets are large in size arising from the amount of drug required in each tablet. A solution to such problems is to formulate the tablets in a form whereby they can be dispersed in water to form a dispersion containing the drug which can then be drunk by the patient.

Known water-dispersible tablets include effervescent formulations which rely on the formation of a gas to quickly break up the tablet, but these involve expensive methods of manufacture and strict regulations for such manufacture. Other known water-dispersible tablets use disintegrating agents such as microcrystalline cellulose used in Feldene (trademark: (™)) dispersible tablets.

According to a first aspect of the invention there is provided a water-dispersible tablet comprising 2% w/w to 90% w/w lamotrigine, and 0.25 to 40% w/w of a pharmaceutically acceptable swellable clay which is present within the granules of the tablet to provide a tablet which is capable of dispersing in water within a period of 3 minutes to provide a dispersion which is capable of passing through a sieve screen with a mesh aperture of 710µ m in accordance with the test for dispersible tablets defined in the British Pharmacopoea 1988, volume II, page 895.

Swellable clays such as Veegum (™) and other magnesium aluminium silicates have previously been studied and proposed for use as disintegrating agents, binders and lubricants in the manufacture of tablets, but such studies and proposals were exclusively with respect to tablets intended for swallowing and not for water-dispersible tablets (Rubenstein, Pharmaceutics - The Science of Dosage Form Design (1990) for disintegrants see p 312 and 314). Moreover, there has never been any suggestion that a clay would be suitable to meet the more stringent requirements for dispersible tablets. Tablets for swallowing need only have a disintegration time in water of less 15 minutes and be able to form particles on disintegration in water that can pass through a 2.00mm mesh aperture (British Pharmacopia test for swallowable tablets). Such long disintegration times and large particle sizes are entirely unsuitable for a dispersible tablet.

Even when swellable clays have been proposed as disintegrating agents for swallowable tablets, they are not regarded as very suitable for such use because their off-white appearance can often discolour the tablet and because they are not as effective as other disintegrating agents (Banker and Anderson - Theory and Practice of Industrial Pharmacy p 328 (1986) and Bhargava et al - Drug Development and Industrial Pharmacy, 17(15), 2093-2102(1991)). In fact, bentonite is identified in Marshall and Rudnic, Modern Pharmaceutics (1990) p 374, as the least swellable of the ten disintegrants listed. There is no mention in the above text-book references of how the swellable clay should be incorporated - i.e. by intra-granular addition or by extra-granular addition. In the former case, the clay would be included in the mixture from which the granulate is formed; in the latter case the clay would be added to the pre-formed granulate.

In J. Pharm. Sci, 55, 1244 (1966), Wai et al. reviewed the following papers relating to swellable clays such as Veegum and bentorite as disintegrating agents: Wai et al., J.Pharm.Sci, 55, 1215(1966); Granberg et al., J.Am.Pharm.Assoc.Sci, 38, 648(1949); Gross et al., J.Am.Pharm.Assoc.Sci, 41, 157(1952); Firouzabadian et al., J.Am.Pharm.Assoc.Sci, 43, 248(1954); Ward et al., Drug Cosmetic Ind, 91, 35(1962); Nair et al., J.Am.Pharm.Assoc.Sci, 46, 131(1957); and Patel et al., Indian J.Pharm., 19, Jan.1957. Wai et al., then compared three grades of Veegum evalulating both extra-granular and intra-granular addition and concluded that "the clays were not good disintegrating agents when wet granulated" (i.e. intra-granular addition), and then went on to recommend extra-granular addition. Furthermore R.T.Vanderbilt and Co. (Manufacturers of Veegum) in their publication "Veegum - The Versatile Ingredient for Pharmaceutical Formulations" at p 19 describe a tablet formulation in which Veegum is added after granulation (tablet No.2). There is no reference in the publication to a formulation of a tablet in which Veegum is added during granulation.

We have found that a swellable clay such as Veegum when added during granulation meets the British Pharmacopoeia (B.P.) standard for dispersible tablets (presently set at a dispersion time of 3 minutes or less).

By using Veegum and other swellable clays in the manner described above, we have been able to prepare water-dispersible tablets containing lamotrigine. The resulting tablets can readily be dispersed in water to form a dispersion which can be drunk by a patient.

The present invention further provides a process for the preparation of a water-dispersible tablet as defined in the first aspect of the invention, said process comprising bringing lamotrigine or a pharmaceutically acceptable salt thereof into association with said swellable clay to form granules, and then compressing the granules to form a tablet which is capable of dispersing in water within a period of 3 minutes to provide a dispersion which is capable of passing through a sieve screen with a mesh aperture of 710µm in accordance with the test for dispersible tablets defined in the British Pharmacopaeia, 1988, volume II, page 895.

Preferably said process comprises the steps of:
a) admixing in dry, finely-divided form lamotrigine or a salt thereof, the swellable clay, optionally with the addition of one or more other pharmaceutical carriers or excipients;
b) addition of a quantity of a pharmaceutically acceptable liquid sufficient to moisten the dry mixture;
c) granulation of the resulting moist mixture with a granulating fluid to form granules;
d) drying the granules and optionally blending the granules with other optional carriers or excipients such as lubricants, glidants, disintegrating agents and flavouring agents; and
e) compression of the granules to form a tablet which is capable of dispersing in water within a period of 3 minutes to provide a dispersion which will pass through a sieve screen with a mesh aperture of 710µm in accordance with the above-defined British Pharmacopoeia test for dispersible tablets.

A tablet according to the invention, as well as being quickly dispersible in water, has the added advantage that it meets the British Pharmacopoeia (B.P.) test for dispersible tablets in respect of dispersion times and dispersion quality (i.e. passage through a 710µm sieve).

Preferably the dispersion time of a tablet according to the invention is less than 2 minutes, more preferably less than 1.50 minutes and most preferably less than 1 minute.

A further advantage of the tablets according to invention is that because a relatively fine dispersion is formed the tablet will have a lower dissolution time and thus the drug may be absorbed into the blood stream much faster. Furthermore the fast dispersion times and relatively fine dispersions obtained with tablets according to the invention are also advantageous for swallowable tablets. Thus tablets according to the invention can be presented both for dispersion in water and also for directly swallowing. Those tablets according to the invention that are intended for swallowing may be film-coated to aid swallowing. Such film-coating however, may increase the dispersion time up to 5 minutes determined in accordance with the above-mentioned B.P. test.

The references herein to tablets according to the invention include both film-coated and non-film-coated tablets.

After the dispersion has passed through the 710µm mesh screen, there should be substantially no residue, except fragments of undissolved tablet coating or shell, remaining on the screen or adhering to the lower surface of the disc, if a disc optionally has been used; and if any residue remains, it should consist of a soft mass having no palpably firm, unmoistened core.

The particle size distribution of the dispersion are set out in the following table with the increasingly preferred values being quoted from left to right.

| Particle Size (µm)* | BP Standard | Preferably | More Preferably | Most Preferably |
|---|---|---|---|---|
| <710 | <100% | 100% | 100% | 100% |
| <300 | - | >50% | >70% | >80% |
| <200 | - | - | >50% | >70% |
| <150 | - | - | - | >50% |

| | | | | |
|---|---|---|---|---|
| * (equivalent spherical volume diameter) | | | | |

Lamotrigine, i.e. 3,5-diamino-6-(2,3-dichlorophenyl)-1,2,4-triazine, and its pharmaceutically acceptable salts which have acceptable dispersibility in water are included in the invention. Thus, for example, a suitable salt of lamotrigine is the isethionate salt (i.e. 2-hydroxymethanesulphonate). It will be appreciated that reference to any active compound also includes any pharmaceutically acceptable salts thereof.

The term "swellable clay" as used herein includes layered clays (such as smectites), porous fibrous clay minerals, and synthetic clay materials related in structure to layered clays and porous fibrous clays.

The term "layered clays" as used herein includes substantially homogeneous layered clays and mixtures thereof, and interstratified or mixed layered clays. Substantially homogeneous layered clays includes the smectite group for example dioctahedral and trioctahedral types. Examples of dioctahedral smectites are the montmorillonite group (montmorillonoids): magnesium and other (e.g. calcium) aluminium silicates such as Veegum in its various grades e.g. Veegum, Veegum HV, Veegum F, and Veegum WG); almasilate; fillers earth (e.g. Surrey finest); American fillers earth; bentonite; beidellite; cheto montmorillonite, Wyoming montmorillonite, Utah montmorillonite; Tatalia and Chambers montmorillonites; and iron rich smectites such as nontrite (e.g. Garfield nontronite) and ferrian smectites.

Examples of triocatahedral smectites (also known as saponites) are Swinefordite, hectorite, stevensite. Examples of smectites containing more unusual elements are Volkhonsite, Medmontite, Sauconite, nickel smectites and vanadium smectites. As well as the montmorillonite group, related smectites such as vermiculites may also have application.

The term "interstratified or mixed layer clays", as used herein includes clays involving different layers arranged in a regular or irregular structure. The most common examples of such clays have generally two components in substantially equal proportions and have been given mineral names such as rectorite (mica-smectite), hydrobiotite (biotite-vermiculite), corrensiten (chlorite-smectite) allettite (talc-saponite). More irregular arrangements include illite-smectite, chlorite-smectite, and kaolinite-smectite. Further examples of interstratified clays are tosudite, tarasovite, allevardite, Japanese bentonite ("acid clays"), AWAZU acid clay, and kaolinite-smectite. Other mixed layer clays may include one or more of the following minerals: clinchlore, chamosite, nimite, thuringite, sudoite, and cookeite. Mixed layer smectities are also known e.g. interdispersed montmorillonite and beidellite layers. The layers of mixed layer clays may be homogeneous or non-homogeneous.

The term "porous fibrous clays" includes polygorskite and sepiolite such as, for example attapulgite and American fuller's earth.

The term "synthetic clay materials" as used herein includes materials related in structure to layered clays and porous fibrous clays such as synthetic hectorite (lithium magnesium sodium silicate) for example laponite(™).

It will be appreciated that within the scope of the invention the following classes of clays have application alone or in combination and in mixed layer clays: kaolinites, serpentines, pyrophyllites, talc, micas and brittle micas, chlorites, smectites and vermiculites, polygorskites and sepiolites. Other phyllosilicates (clay minerals) which may be employed in the tablets according to the invention are allophane and imogolite.

The following references describe the characterisation of clays of the above types: Chemistry of Clay and Clay Minerals. Edited by A.C.D. Newman. Mineralogical Society Monograph No. 6, 1987, Chapter 1; S.W. Bailey; Summary of recommendations of AIPEA Nomenclature Committee, Clay Minerals 15, 85-93; and A Handbook of Determinative Methods in Mineralogy, 1987, Chapter 1 by P.L. Hall.

Suitably the swellable clay is a pharmaceutically acceptable crystalline mineral clay having a lattice structure which expands upon hydration, preferably a pharmaceutically acceptable smectite or attapulgite clay, especially a montmorillonoid, more preferably yet a montmorillonoid chosen from the group consisting of montrnorillonite, sauconite, vermiculite, bentonite and hectorite, still more preferably an aluminium magnesium silicate and most preferably Veegum (™).

The term "smectite" as used herein in relation to tablets of the present invention includes the smectites as exemplified herein and with reference to O'Brian P. and Williamson C.J., in "Clays and Clay Minerals vol. 38 No. 3 pp322-326, 1990" and the other clay nomenclature references set out hereinbefore.

The term "magnesium aluminium silicate" as used herein in relation to tablets of the present invention should be understood to include the Aluminium Magnesium Silicate defined in the British Pharmacopoeia, volume 1, pages 27-28, 1988 and the Magnesium Aluminium Silicate defined in the United States Pharmacopoeia, National Formulary XVI, pages 1943-1944, 1990. Advantageously, said silicate is in the form of a microfine powder having a No. 325 US Standard mesh particle size, a viscosity of 250 cps (± 25%) for a 5.5% (w/v) aqueous dispersion and an acid demand (the volume in ml. of O.lN hydrochloric acid required to reduce the pH of one gram to 4) of 6-8: such a material is available as VEEGUM F (R.T. Vanderbilt Co., New York, N.Y., U.S.A.; K & K-Greeff Chemicals Ltd., Croydon, Surrey CR9 3QL, England).

For a dispersible tablet containing lamotrigine, the intra-granular amount of swellable clay such as a crystalline mineral clay for example, magnesium aluminium silicate is suitably present in the following general ranges 0.25 to 60% w/w, preferably 0.25 to 50% w/w, more preferably 0.5 to 50% w/w, more preferably still 1 to 50% w/w, more preferably still 1 to 40% w/w, more preferably still 2 to 20% w/w, more preferably still 2.5 to 20% w/w, still more preferably 1 to 10% w/w, still more preferably 3 to 10% w/w, and most preferably 5 to 10%, most desirably about 5% w/w.

The tablets according to the invention conveniently contain 2.5 to 500 mg. desirably 5 to 250 mg. of lamotrigine calculated as lamotrigine base. Preferred said unit doses include 5 mg., 12.5 mg., 25 mg., 50 mg., 100 mg., 150 mg., 200 mg. and 250 mg., calculated as the base. For tablets having a total weight of about 55 to 65mg and containing about 5mg lamotrigine, the swellable clay, e.g. Veegum F, is preferably present in an amount of 2 to 4mg, especially about 3mg. Similarly for a tablet having a weight of about 220 to 350mg and containing about 80 to 120mg, preferably 100mg of lamotrigine, the swellable clay, e.g. Veegum F, is preferably present in amount of 5 to 20mg, especially about 12mg.

In general the tablets according to the invention contain lamotrigine in the following percentage proportions - Lamotrigine - 3 to 90% w/w, preferably 5 to 40% w/w.

The said lamotrigine dispersible tablets may be employed in human medicine in the treatment of disorders of the central nervous system and in particular in the treatment of epileptic seizures. They may be administered one or more times per day, for example up to five times per day, at the discretion of the attendant physician and dependent upon the age and condition of the patient, the particular disorder being treated, the unit dose adopted and the total dose required. A suitable daily dose for the treatment of epileptic seizures will generally lie in the range of 5 to 500 mg., more often in the range of 25 to 400 mg., calculated as the base.

The physical size of the said tablets is desirably such as to permit their dispersion, prior to oral ingestion, in an acceptably small volume of water. Thus, for example, a tablet containing 5 mg. (calculated as the base) of lamotrigine or a salt thereof, a dose especially suitable for paediatric use, is advantageously small enough to disperse in the volume of water held in a standard 5 ml. medicine spoon.

Tablets of the invention containing lamotrigine (or a salt thereof) advantageously include a magnesium aluminium silicate such as Veegum F as the swellable clay together with further optional pharmaceutical carriers or excipients referred to above such as binders, lubricants, fillers, disintegrating agents etc.

A tablet according to the invention should desirably have a friability of about 2% or less, preferably 0.5% or less.

Based on experiments that we have carried out, it has been found that in addition to the amount of swellable clay present within the granules of the tablet, a farther amount of swellable clay may be present outside the granules. At very low intra-granular amounts (such as 1% w/w or below), higher extra-granular amounts (such as about 10% w/w or more) may decrease the dispersion time, but in general extra-granular addition has little or no effect on the dispersion time. The maximum percentage(s) of the clay present within the granules and, optionally outside the granules, may be limited by other practical considerations such as poor flow and compression properties.

Other excipients suitable for inclusion in the tablets according to the invention include the following:
a) Binders and Adhesives: we have found that if there is sufficient amount of swellable clay such as Veegum F present within the granules, then a separate binder is not required (i.e. the clay also acts as a binder). Preferably however a separate binder is present in a sufficient amount to provide a tablet having a satisfactory tablet hardness and satisfactory dispersion characterstics. The amount of binder will vary depending on the overall tablet formulation and type of binder used but general functional limits for most tablets of the invention are 0 to 25% w/w. The following binders and amounts are suitable for inclusion in a tablet according to the invention. The concentration of the binder in the granulation fluid (% w/v) is given (% w/w in tablet will vary according to the volume of granulating solution used to form a satisfactory tablet): Examples of binders are: acacia mucilage 0 to 25% w/v, preferably 1 to 5% w/v, alginic acid 0 to 20.0% w/v, preferably 1 to 5% w/v, polyvinylpyrrolidone (povidone) 0 to 15.0% w/v, preferably 0.5 to 5% w/v, gelatin 0 to 20.0% w/v, preferably 1 to 5.0% w/v, sucrose 0 to 70.0% w/v, preferably 2.0 to 20.0% w/v, starch mucilage 0 to 10.0% w/v, preferably 0.5 to 5.0% w/v, pregelatinised starch 0 to 10.0% w/v, preferably 0.5 to 5.0% w/v, starch paste 0 to 10.0% w/v, preferably 5.0 to 10.0% w/v, sodium alginate 0 to 5.0% w/v, preferably 1.0 to 3.0% w/v, sorbitol 0 to 10.0% w/v, preferably 3.0 to 10.0% w/v, tragacanth 0 to 20% w/v, preferably 5.0 to 10.0% w/v, glucose 0 to 50%, preferably 5 to 25% w/v, hydroxypropylmethyl cellulose (HPMC) 0 to 10% w/v, preferably 1.0 to 5.0% w/v, magnesium aluminium silicate 0 to 40% w/v, preferably 2 to 10% w/v, starch paste 0 to 25% w/v, preferably 5 to 15% w/v, polyvinylpyrrolidone 0 to 15% w/v, preferably 3 to 10% w/v, carboxymethylcellulose sodium 0 to 10% w/v, preferably 1 to 6% w/v, dextrin 0 to 50% w/v, preferably 5 to 25% w/v, ethyl cellulose 0 to 10% w/v, preferably 1 to 6% w/v, polyethylene glycol 0 to 5% w/v, guar gum 0 to 10% w/v, preferably 1 to 5% w/v, zein 0 to 30% w/v, preferably 1 to 10% w/v, hydroxyethyl cellulose 0 to 5% w/v, preferably 2 to 4% w/v, hydroxypropyl cellulose up to 5% w/v, preferably 2 to 4% w/v, methyl cellulose up to 20% w/v, preferably 1 to 10% w/v, polymethacrylates up to 25% w/v, preferably 5 to 10% w/v, carboxymethylcellulose calcium 0 to 20% w/v, preferably 5 to 10% w/v.
b) Examples of suitable disintegrating agents which can be incorporated into a tablet according to the invention are: microcrystalline cellulose (e.g. Avicel (™)) 0 to 30% w/w, preferably 5 to 10% w/w, sodium carboxymethyl cellulose (e.g. Nymcel (™)) 0 to 5% w/w, preferably 1 to 2% w/w, calcium carboxymethyl cellulose 0 to 20% w/w, preferably 1 to 5% w/w, modified cellulose gum (e.g. Ac-Di-Sol (™)) 0 to 10% w/w, preferably 1 to 5% w/w, cross-linked povidone 0 to 10% w/w, preferably 2 to 6% w/w, alginic acid and alginates 0 to 10% w/w, 2 to 5% w/w, pregelatinised starch 0 to 10% w/w, preferably 0.5 to 5% w/w, sodium starch glycollate (e.g. Explotab (™), Primojel (™)) 0 to 10% w/w, preferably 0.5 to 5% w/w, modified corn starch (e.g. starch 1500 R) 0 to 20% w/w, preferably 1 to 10% w/w, starch (e.g. potato/maize starch ) 0 to 15% w/w, preferably 0.2 to 10% w/w, ion exchange resin such as polacrin potassium (e.g. Amberlite IRP-88) up to 5% w/w, preferably 0.5 to 2.0% w/w.
   Work with lamotrigine is supportive of the view that if low hydroxypropyl cellulose (LHPC) is used a suitable dispersion can be obtained without the need for a separate wetting agent/surfactant.
c) Fillers: These serve the purpose of bulking up the tablet to a suitable size and aiding compressibility especially in lower dosage tablets. The amount of filler depends on its type, size of tablet and amount of active compound. When the concentration of active compound is below 60% w/w, more preferably 45% w/w and most preferably below 30% w/w, an inorganic water-insoluble filler is advantageously used. Examples of water-soluble fillers (which can be used in general quantities of 0 to 95% w/w) are: soluble lactose, compressible sugar, confectioners sugar, dextrose, mannitol, sodium chioride, sorbitol, xylitol, sodium chloride F. Examples of water-insoluble fillers (which can be used in general quantities of 0 to 93% w/w) are: calcium carbonate, magnesium carbonate, calcium phosphate (e.g. di and tri basic calcium phosphate), calcium sulphate, kaolin, microcrystalline cellulose, powdered cellulose, pregelatinized starch 5 to 75%, starch, barium sulphate, magnesium trisilicate, aluminium hydroxide.
   Inclusion of a filler having a negative heat of solution in water, for example marinitol, sorbitol and xylitol, provides tablets which, in addition to being water-dispersible, are especially suitable for chewing in the mouth, the dissolving of such an excipient in the saliva producing a cool, pleasant sensation.
d) Lubricants: Generally lubricants are used in as low an amount as possible. Examples of lubricants with percentage weights which are suitable for a tablet are: stearates (e.g. magnesium or calcium stearate) 0.2 to 5% w/w, preferably 0.25 to 1% w/w, talc 0.19 to 5% w/w, preferably 1 to 2% w/w, polyethylene glycol 0.19 to 5% w/w, preferably 2 to 5% w/w, liquid paraffin 0.18 to 5% w/w, preferably 2 to 5% w/w, sodium lauryl sulphate 0.19 to 5% w/w, preferably 0.5 to 2% w/w, magnesium lauryl sulphate 0.12 to 5% w/w, preferably 1 to 2% w/w, colloidal silicon dioxide 0.1 to 5% w/w, preferably 0.1 to 1.0% w/w, palmitostearate 0.01 to 5% w/w, preferably 1 to 3% w/w, stearic acid 0.01 to 5% w/w, preferably 1 to 3% w/w, zinc stearate 0.01 to 2% w/w, 0.5 to 1.5% w/w, hydrogenated vegetable oil 0.5 to 5% w/w, preferably 1 to 3% w/w. More suitably the lower value is 0.25%.
e) Wetting agents/surfactants: examples with suitable amounts are: sodium dodecyl sulphate 0 to 10% w/w, preferably 0.5 to 2% w/w, sodium lauryl sulphate 0 to 10% w/w, preferably 0.1 to 3.0% w/w, polyoxyethylene sorbitan fatty acid esters (Tweens) 0 to 3% w/w, preferably 0.05 to 1.0% w/w, polyoxyethylene stearates 0 to 2% w/w, preferably 0.05 to 1.0% w/w, sorbitan fatty acid esters (Spans) 0 to 3% w/w, preferably 0.05 to 1.0% w/w.
f) Glidants: for example, talc 0 to 5% w/w, preferably 1 to 2% w/w, starch 0 to 15% w/w, preferably 2 to 10% w/w, magnesium stearate up to 5%, preferably 0 - 2.0% w/w, silica derivatives generally 0 to 1% w/w, preferably 0.2 to 0.5% w/w, such as colloidal silica (e.g. Aerosil) 0 to 0-5% w/w, preferably 0.25 to 3% w/w, pyrogenic silica 0 to 2% w/w, preferably 0.25 to 1% w/w, hydrated sodium silicoaluminate 0 to 2% w/w, preferably 0.5 to 1% w/w, colloidal silicon dioxide 0 to 0.5% w/w.
g) Flavouring agents: are used in for example approximate quantities of 0 to 5% w/w, preferably 0.25 to 2% w/w, orange, cherry and strawberry, raspberry, grape and passion fruit.
h) Sweetening agents: for example sodium saccharin 0 to 10% w/w, preferably, 0.5 to 5.0% w/w, aspartame 0 to 10% w/w, preferably 0.25 to 5.0% w/w, confectioners sugar 0 to 30% w/w, preferably 5 to 20% w/w, sorbitol 25 to 90% w/w, preferably 0.5 to 10% w/w, sucrose 0 to 85% w/w, preferably 0.5 to 20% w/w, xylitol 0 - 20% w/w, preferably 0.5 to 10% w/w.

Such materials may be incorporated at the appropriate stage(s) of the manufacturing process together with any other agents (e.g. colourants).

Other aspects of the tablet preparation will now be discussed.

Suitably the dry mixing is effected with a mixing time of 5 minutes to 25 minutes preferably about 10 minutes.

The swellable clay can be dry mixed with lamotrigine and other excipients and then granulating solution added, or the clay and other excipients can be dispersed firstly in the granulating solution and then added to lamotrigine and any other excipients prior to granulation.

The liquid employed to moisten the dry mixture, prior to the granulation step, is preferably aqueous, for example water or a mixture of water and a suitable alcohol such as ethanol or isopropanol.

Wet mixing or granulating times which are suitable (depending on the type of mixer used) are 5 to 20 minutes.

Suitable granule drying times and conditions (which will vary according to the type of equipment used and batch size of granules) are about 50 to 80°C, (using a dryer such as with a tray or fluid bed dryer) to obtain a moisture content generally below about 4%.

Generally suitable compression weights and final table hardness will vary according to the size of tablet, but generally suitable values are as follows:

| Approximate Tablet weight (mg) | Approximate Tablet diameter (mm) | Approximate Target tablet hardness (Kp) |
|---|---|---|
| 60 | 5.6 | 1-2 |
| 80 | 6.4 | 3-4 |
| 125 | 7.4 | 4-5 |
| 250 | 8.6 | 5-6 |
| 330 | 9.4 | 6-8 |
| 500 | 11.0 | 10-12 |
| 600 | 11.8 | 10-14 |
| 1000 | 14.0 | 12-16 |

The tablets may optionally be film-coated, for example with hydroxypropylmethyl cellulose, polyethylene glycol or titanium dioxide, and/or may be scored and/or may be polished, for example with polyethylene glycol 8000. If the tablets are film-coated, this makes them easier to swallow or chew (i.e. the tablets are suitable for either dispersion in water or for direct swallowing or chewing), but the dispersion time is increased.

The present invention also provides:
a) Granules, suitable for use in the preparation of a water-dispersible compressed tablet as defined hereinbefore, comprising lamotrigine or a pharmaceutically acceptable salt thereof together with a pharmaceutically acceptable crystalline mineral clay as dispersing agent;
b) Use of granules as defined above in the preparation of a water-dispersible compressed tablet as defined hereinbefore optionally comprising the addition of a farther amount of crystalline mineral clay compound after granulation and before compression;
c) A water-dispersible tablet as defined hereinbefore comprising lamotrigine or a pharmaceutically acceptable salt thereof together with a pharmaceutically acceptable crystalline mineral clay having a lattice structure which expands upon hydration as dispersing agent and an additional pharmaceutically acceptable disintegrating agent. The lamotrigine or a pharmaceutically acceptable salt thereof together with the mineral clay and additional disintegrating agent are comprised within the tablet in granulated form.
d) A method for the preparation of a lamotrigine water-dispersible tablet which comprises the steps of
   admixture in dry, finely-divided form of lamotrigine or a pharmaceutically acceptable salt thereof, a pharmaceutically acceptable crystalline mineral clay which may be chosen from the group consisting of attapulgite, smectite and montmorillonoid clays or magnesium aluminium silicate, and an additional disintegrating agent;
   optional addition of other pharmaceutical ingredients; addition of a quantity of a pharmaceutically acceptable liquid sufficient to moisten the mixture;
   granulation of the resulting moist mass;
   drying of the granules and blending of the granules with optional lubricants, glidant, flavours, etc., and
   formation of the blend into a tablet.

Especially preferred tablets are those wherein the lamotrigine is present as the base.

A suitable formulation of a dispersible tablet containing 25 to 200mg lamotrigine would be:

| | |
|---|---|
| Lamotrigine | 30% w/w to 50% w/w, preferably 35-45% |
| Calcium carbonate | 26% w/w to 46% w/w, preferably 31-41% |
| LHPC-LH11 or microcrystalline cellulose (e.g. Avicel PH101) | 5% w/w to 30% w/w, preferably 5-15% |
| Magnesium aluminium silicate (Veegum F) or bentonite | 0.25% w/w to 30% w/w, preferably 0.25-10% |
| Povidone | 0.25% w/w to 5.0% w/w, preferably 0.5-2% |
| or pregelled starch | 1.0% w/w to 8.0% w/w, preferably 2-5% |
| Sodium starch glycollate | 0% w/w to 8% w/w, preferably 0-5% |
| Magnesium stearate | 0.25% w/w to 2% w/w, preferably 0.25-1% |

and if optionally film coated:

| | |
|---|---|
| Opadry | 0.1% w/w to 2% w/w, preferably 0.25-1% |
| Polyethylene glycol 8000 | 0.1% w/w to 0.5% w/w, preferably 0.1-0.2% |

A suitable formulation of a dispersible tablet containing 5mg to 50mg of lamotrigine would be as follows, (values being in % w/w).

| | |
|---|---|
| Lamotrigine | 3-13 preferably 5-11 |
| Lactose or calcium carbonate | 50-60 preferably 53-59 |
| Microcrystalline cellulose (e.g. Avicel PH101) or LHPC-LH11 | 20-35 preferably 24-30 |
| Sodium starch glycollate | 0-8 preferably 0-5 |
| Magnesium aluminium silicate (Veegum F) or bentonite | 0.25-30 preferably 0.25-10 |
| Povidone K30 | 0.25-5.0 preferably 0.5-2.0 |
| or pregelled starch | 1.0-8.0 preferably 2-5 |
| Sodium docusate | 0-0.5 preferably 0.5-0.15 |
| Sodium saccharine | 0-3 preferably 0.5-2 |
| Magnesium stearate | 0.25-2 preferably 0.25-1 |

and if optionally film coated

| | |
|---|---|
| Opadry | 0.1-2.0 preferably 0.25-1 |
| Polyethylene glycol 8000 | 0.1-0.5 preferably 0.1-0.2 |

| Examples 1-9 describe the preparation of lamotrigine tablets according to the invention. | | | | | | | | | |
|---|---|---|---|---|---|---|---|---|---|
| Example Number | 1 mg/tablet | 2 mg/tablet | 3 mg/tablet | 4 mg/tablet | 5 mg/tablet | 6 mg/tablet | 7 mg/tablet | 8 mg/tablet | 9 mg/tablet |
| Lamotrigine | 100 | 5.0 | 5.0 | 100 | 100 | 100 | 100 | 100.0 | 100.0 |
| Calcium carbonate | 95 | NIL | NIL | NIL | 95 | NIL | NIL | 95.0 | 90.0 |
| Lactose | NIL | 34 | 35.0 | 15 | NIL | 98.1 | 84 | | |
| L HPC-LH11 | 25 | NIL | NIL | NIL | 25 | NIL | NIL | 25.0 | 25.0 |
| Veegum F | 12 | 3.0 | 3.0 | 7.5 | 12.0 | 16.0 | 12 | 12.0 | 12.0 |
| Povidone K30 | 3.0 | 0.6 | 0.6 | 1.5 | 3.0 | 3.2 | 3 | 3.0 | 3.0 |
| Explotab | 10.0 | 2.0 | 1.2 | 6.0 | NIL | 12.8 | 10.0 | - | 10.0 |
| Sodium Saccharin | 2.5 | 0.5 | 0.5 | NIL | NIL | NIL | NIL | - | - |
| Aspartame | NIL | NIL | NIL | 4.0 | 7.5 | NIL | 7.5 | 7.5 | 7.5 |
| Microcrystalline cellulose (Avicel PH101) | NIL | 17 | 17 | 15 | NIL | 89.6 | 23 | - | - |
| Sodium docusate | NIL | 0.05 | NIL | NIL | NIL | 0.26 | 0.2 | - | - |
| Magnesium stearate | 2.5 | 0.4 | 0.4 | 1.5 | 2.5 | 3.2 | 2.5 | 2.5 | 2.5 |
| Tablet weight (mg) | 250 | 62.55 | 62.70 | 150.5 | 245 | 323.16 | 242.2 | 245.0 | 251.24 |

### Method of Preparation

The tablets described in Examples 1-9 above were prepared according to the following general method:
(a) A dry mixture was made of all components except Povidone/PVP K30, sodium docusate (if present) and magnesium stearate;
(b) The Povidone/PVP K30 and sodium docusate (if present) were dissolved in 50% aqueous alcohol to form a granulation solution;
(c) The granulation solution was added to the dry mixture to form granules;
(d) The wet granules were dried in a fluid bed dryer;
(e) The granules were then sifted through a 1000µm diameter mesh sieve; and
(f) The dried granules were blended with the magnesium stearate and compressed to form tablets.

Flavouring agents where present were added at blending step (f) above.

This general method is illustrated with respect to the following specific examples.

### Uncoated Tablets

(a) A dry mixture was made of all components except Povidone/PVP K30 and magnesium stearate using a Diosna P100 (high shear mixer - granulator) for 3 minutes.
(b) The Povidone/PVP K30 was dissolved in 50% aqueous alcohol to form a granulation solution.
(c) The granulation solution was added to an approximate quantity of 300ml per kg dry weight to the dry mixture to form granules. Wet mixing was carried out for approximately 5 minutes.
(d) The wet granules were dried in an Aeromatic T3 fluid bed drier at a temperature of 70°C for approximately 30 minutes. The moisture content of the granules was approximately 4%.
(e) The granules were then sifted through a 1000µm diameter mesh sieve using a Jackson Crockatt No.7 sifter.
(f) The dried granules were blended with the magnesium stearate using a collette mixer for approximately 10 minutes and compressed to form tablets.

### Example 2

(a) A dry mixture was made of all components except Povidone/PVP K30 and magnesium stearate using a Z-blade Morton Mixer, mixing for 10 minutes at a slow speed.
(b) The Povidone/PVP K30 was dissolved in 50% aqueous alcohol to form a granulation solution;
(c) The granulation solution was added to an approximate quantity of 350ml per kg dry weight to the dry mixture to form granules;
(d) Wet mixing was carried out for approximately 10 minutes. The wet granules were sieved through a 2000µm mesh sieve;
(e) The wet granules were dried in an Aeromatic fluid bed drier at a temperature of 70°C for approximately 25 minutes;
(f) The granules were then sifted through a 1000µm diameter mesh sieve;
(g) The dried granules were blended with the magnesium stearate using a Rotomixer rotary blender for 5 minutes and compressed to form tablets using a Manesty D3 Rotary press fitted with 5.6mm diameter round (normal curvature) punches and dies. Tablets were compressed to a weight of 62.55mg ± 2%.

Flavouring agents may be added at blending step (g) above.

For a 50mg tablet, the same procedure was used, except that a die of 11.8mm diameter was used and the tablets were compressed to a weight of 625.5mg ± 2%.

The lamotrigine tablets could be optionally film coated using the same procedure as described above.

The tablets prepared in accordance with the above Examples were then tested as follows.

### Tablet Evaluation Methods

1. Average tablet weight. Twenty tablets were weighed on an analytical balance and the average tablet weight calculated.
2. Tablet breaking strength (kilo pond-kp). 5 tablets were individually tested using a Schleuniger crushing strength tester, and the average breaking strength calculated.
3. Friability (% loss). 10 tablets, accurately weighed, were subjected to 10 minutes friability testing using a Roche Friabilator. The tablets were dedusted, reweighed, and the weight loss due to the friability was calculated as a percentage of the initial weight.
4. Dispersion Disintegration time DT (BP 1988). 6 tablets were tested in accordance to the above-defined BP test (without discs) for dispersible tablets. This utilises water at a temperature of 19-21°C.
5. Dispersion Quality. In accordance with the BP uniformity of dispersion test for dispersible tablets (BP 1988 Volume II page 895), two tablets were placed in 100ml of water at 19-21°C and allowed to disperse. A smooth dispersion was produced which passed through a 710µm mesh sieve.Granule Evaluation Methods
1. Loss on Drying (LOD). The residual moisture content of the granule (LOD) was determined on a 3-4g sample using a Computrac moisture analyser set to 90°C operated in accordance with the manufacturer's procedure.
2. Weight Median Diameter (WMD). A 10g sample of granule was sifted for 2 minutes at suitable pulse and sift amplitudes in an Allen Bradley sonic sifter in accordance with manufacturer's instructions. Sieves of 710µm, 500µm, 355µm, 250µm, 150µm, 106µm and 53µm were used. The WMD was calculated from the cumulative percentage undersize size distribution using a computer programme.

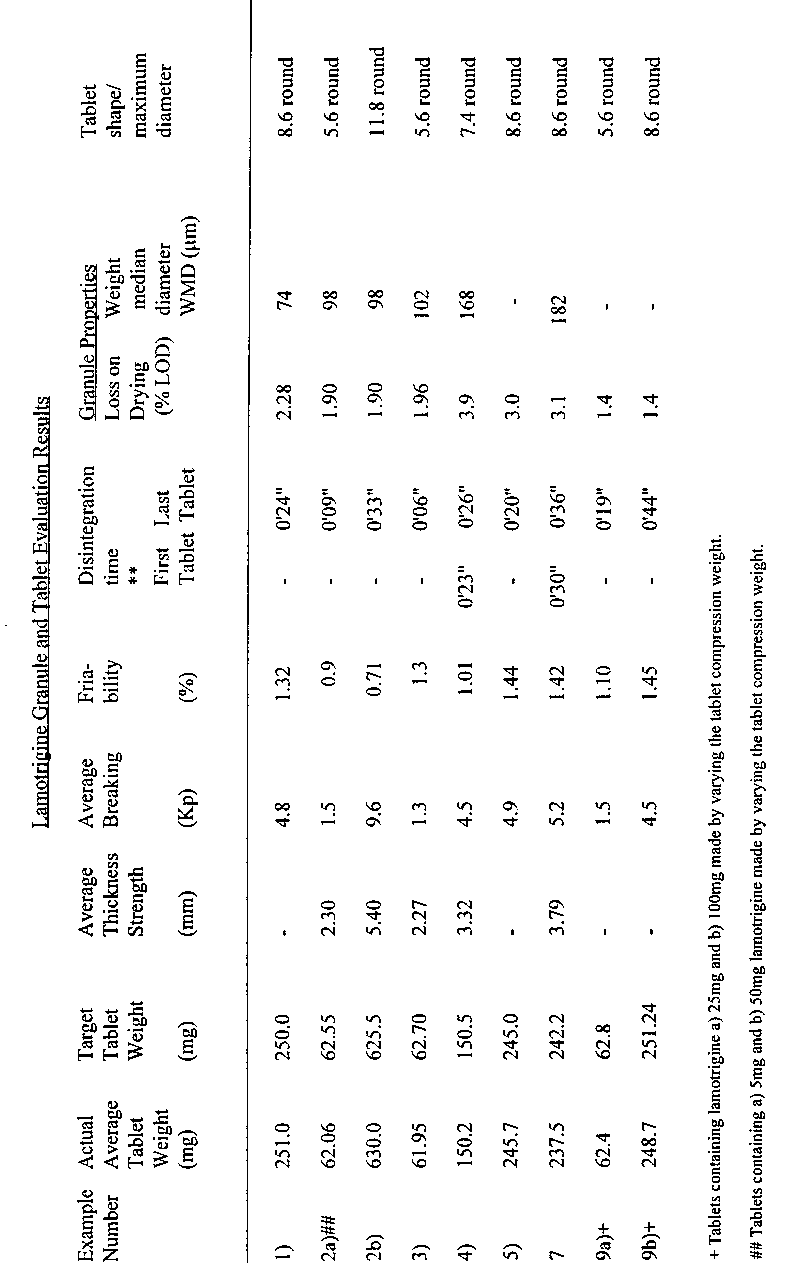

The particle size distribution was determined using a Malvern 2600 particle analyser as follows. The instrument was set to analyse particles in liquid with magnetic stirrer fitted. A 300mm focal length lens was used.
1. Disperse tablet in 100ml of de-ionised water.
2. Agitate solution for approximately 2 hours.
3. Filter or centrifuge solution to obtain liquor which should be saturated with all ingredients present in the tablet.
4. Disperse second tablet in 50ml of saturated liquor allowing 3 minutes to fully disperse. Agitate vigorously and remove a sample of the dispersion within 5 minutes adding sufficient quantity to the Malvern PIL cell containing the liquor to obtain an observation value of 0.15-0.30. Analyse sample.

## Claims

1. A water-dispersible tablet comprising 2% w/w to 90% w/w lamotrigine or a pharmaceutically acceptable salt thereof, and 0.25 to 40% w/w of a pharmaceutically acceptable swellable clay which is present within the granules of the tablet to provide a tablet which is capable of dispersing in water within a period of 3 minutes to provide a dispersion which is capable of passing through a sieve screen with a mesh aperture of 710µm in accordance with the test for dispersible tablets defined in the British Pharmacopoeia 1988, volume II, page 895.

2. A tablet as claimed in claim 1 wherein the swellable clay is a smectite or attapulgite.

3. A tablet as claimed in claim 2 wherein the smectite is selected from the montmorillonoid group.

4. A tablet as claimed in claim 3 wherein the montmorillonoid group is selected from the group consisting of montmorillonite, seuconite, verniculite, bentonite, hectorite and aluminium magnesium silicate.

5. A tablet as claimed in claim 4 wherein the montmorillonoid is magnesium aluminium silicate or bentonite.

6. A tablet as claimed in claim 5 wherein the magnesium aluminium silicate is veegum F.

7. A tablet as claimed in any one of the preceding claims wherein the swellable clay is present within the granules of the tablet in an amount of 1 to 40% w/w.

8. A tablet as claimed in claim 7 wherein the swellable clay is present in an amount of 1 to 10% w/w.

9. A tablet as claimed in claim 8 wherein the swellable clay is present in an amount of 5 to 10% w/w.

10. A tablet as claimed in any one of the preceding claims which further comprises a disintegrating agent.

11. A tablet as claimed in any one of the preceding claims wherein the additional disintegrating agent is sodium starch glycollate or lowhydroxypropylcellulose.

12. A tablet as claimed in any one of the preceding claims which further comprises a binder.

13. A tablet as claimed in claim 12 wherein the binder is povidone k30.

14. A tablet as claimed in any one of the preceding claims which farther comprises a filler.

15. A tablet as claimed in any one of claims 1 to 14 which is capable of dispersing in water within a period of 2 minutes.

16. A tablet as claimed in any one of the preceding claims wherein the amount of lamotrigine is 5 to 250mg.

17. A tablet as claimed in any one of the preceding claims wherein the dispersion contains particles having a particle size distribution of 100% less than 710µm, and more than 50% less than 300µm.

18. A tablet as claimed in claim 17 wherein the dispersion contains particles having a particle size distribution of 100% less than 710µm, more than 70% less than 300µm, and more than 50% less than 200µm.

19. A tablet as claimed in any one of the preceding claims wherein the tablet contains 25 to 200mg lamotrigine or a salt thereof and comprises a formulation of 30% w/w to 50% w/w lamothgine or a salt thereof, 26% w/w to 46% w/w calcium carbonate, 5 to 30% w/w lowhydroxypropylcellulose-LH11 or microcrystalline cellulose, 0.25% w/w to 30% w/w magnesium aluminium silicate or bentonite, 0.25 to 5% w/w povidone or 1 to 8% w/w pregelled starch, 0 to 8% w/w sodium starch glycollate, 0.25% w/w to 2% w/w magnesium stearate, and optional film coating composites of 0.1% w/w to 2% w/w opadry and 0.1% w/w to 0.5% w/w polythylene glycol.

20. A tablet as claimed in claim 19 wherein the formulation is 35% w/w to 45% w/w lamotrigine or a salt thereof, 31% w/w to 41% w/w calcium carbonate, 5 to 15% w/w lowhydroxypropylcellulose or microcrystalline cellulose, 0.25% w/w to 10% w/w magnesium aluminium silicate or bentonite, 0.5 to 2% w/w povidone or 2 to 5% w/w pregelled starch, 0 to 5% w/w sodium starch glycollate, 0.25% w/w to 1% w/w magnesium stearate, and optional film coating composites of 0.25% w/w to 1% w/w opadry and 0.1% w/w to 0.2% w/w polythylene glycol.

21. A tablet as claimed in any one of claims 1 to 17 wherein the tablet contains 5mg to 50mg lamotrigine or a salt thereof and the formulation comprises 3% w/w to 13% w/w lamotrigine or a salt thereof, 50% w/w to 60% w/w lactose or calcium carbonate, 20% w/w to 35% w/w lowhydroxypropylcellulose or microcrystalline cellulose, 0 to 8% w/w sodium starch glycollate, 0.25% w/w to 30% w/w magnesium aluminium silicate or bentonite, 0.25% w/w to 5% w/w povidone or 1% w/w/ to 8% w/w/ pregelled starch, 0 to 5% w/w sodium docusate, 0 to 3% sodium saccharine. 0.25% w/w to 2% w/w magnesium stearate, and optional film coating composites of 0.1% w/w to 2% w/w opadry, and 0.1% w/w to 0.5% w/w polyethylene glycol.

22. A tablet as claimed in claim 21 wherein the formulation is 5% w/w to 11% w/w lamottigine or a salt thereof, 53% w/w to 59% w/w lactose or calcium carbonate, 24% w/w to 30% w/w lowhydroxypropylcellulose or microcrystalline cellulose, 0 to 5% w/w sodium starch glycollate, 0.25% w/w to 10% w/w magnesium aluminium silicate or bentonite, 0.5% w/w to 2% w/w povidone or 2% w/w to 5% w/w pregelled starch, 0.15% w/w to 0.5% w/w sodium docusate, 0.5% w/w to 2% sodium saccharine. 0.25% w/w to 1% w/w magnesium stearate, and optional film coating composites of 0.25% w/w to 1% w/w opadry, and 0.1% w/w to 0.2% w/w polyethylene glycol.

23. A process for the preparation of a water-dispersible tablet comprising 2% to 90% w/w lamotrigine or a pharmaceutically acceptable salt thereof 0.25 to 40% of a w/w of a pharmaceutically acceptable swellable clay; said process comprising bringing lamotrigine into association with said swellable clay to form granules, and then compressing the granules to form a tablet which is capable of dispersing in water within a period of 3 minutes to provide a dispersion which is capable of passing through a sieve screen with a mesh aperture of 710µm in accordance with the test for dispersible tablets defined in the British Pharmacopoeia, 1988, volume II, page 895.

24. A process as claimed in claim 23 comprising the steps of:
a) admixing in dry, finely-divided form lamotrigine or a salt thereof and the swellable clay, optionally with the addition of one or more other pharmaceutical carriers or excipients;
b) addition of a quantity of granulation fluid sufficient to moisten the dry mixture;
c) granulation of the resulting moist mixture to form granules;
d) drying the granules and optionally blending the granules with other optional carriers or excipients such as lubricants, glidants, and flavouring agents; and
e) compression of the granules to form a tablet which is capable of dispersing in water within a period of 3 minutes to provide a dispersion which will pass through a sieve screen with a mesh aperture of 710µm in accordance with the above-defined British Pharmacopoeia test for dispersible tablets.

25. A process as claimed in claims 23 or 24 wherein a binder is added to the lamotrigine or a salt thereof and swellable clay.

26. A process as claimed in any one of claims 23 to 25 wherein a disintegrating agent is added to the lamotrigine or a salt thereof and swellable clay.

27. A process as claimed in any one of claims 23 to 26 wherein a filler is added to the lamotrigine or a salt thereof and swellable clay.

## Patentansprüche

1. Wasserdispergierbare Tablette, umfassend 2 % G/G bis 90 % G/G Lamotrigin oder ein pharmazeutisch akzeptables Salz davon und 0,25 bis 40 % G/G eines pharmazeutisch akzeptablen quellbaren Tons, der innerhalb der Granalien der Tablette vorliegt, um eine Tablette bereitzustellen, die in Wasser innerhalb eines Zeitraums von 3 min zur Bereitstellung einer Dispersion dispergierbar ist, die durch einen Siebfilter mit einer Maschenweite von 710 µm gemäß dem in der Britischen Pharmakopoe 1988, Band II, Seite 895 definierten Test für dispergierbare Tabletten gelangen kann.

2. Tablette gemäß Anspruch 1, worin der quellbare Ton ein Smektit oder Attapulgit ist.

3. Tablette gemäß Anspruch 2, worin der Smektit ausgewählt ist aus der Montmorillonoid-Gruppe.

4. Tablette gemäß Anspruch 3, worin die Montmorillonoid-Gruppe ausgewählt ist aus der Gruppe, bestehend aus Montmorillonit, Seukonit, Vermiculit, Bentonit, Hektorit und Aluminiummagnesiumsilicat.

5. Tablette gemäß Anspruch 4, worin der Montmorillonoid Magnesiumaluminiumsilicat oder Bentonit ist.

6. Tablette gemäß Anspruch 5, worin das Magnesiumaluminiumsilicat Veegum F ist.

7. Tablette gemäß einem der vorhergehenden Ansprüche, worin der quellbare Ton innerhalb der Granalien der Tablette in einer Menge von 1 bis 40 % G/G vorliegt.

8. Tablette gemäß Anspruch 7, worin der quellbare Ton in einer Menge von 1 bis 10 % G/G vorliegt.

9. Tablette gemäß Anspruch 8, worin der quellbare Ton in einer Menge von 5 bis 10 % G/G vorliegt.

10. Tablette gemäß einem der vorhergehenden Ansprüche, die zusätzlich ein Sprengmittel umfaßt.

11. Tablette gemäß einem der vorhergehenden Ansprüche, worin das zusätzliche Sprengmittel Natriumstärkeglykolat oder Niederhydroxypropylcellulose ist.

12. Tablette gemäß einem der vorhergehenden Ansprüche, die zusätzlich ein Bindemittel umfaßt.

13. Tablette gemäß Anspruch 12, worin das Bindemittel Povidon K30 ist.

14. Tablette gemäß einem der vorhergehenden Ansprüche, die zusätzlich einen Füllstoff umfaßt.

15. Tablette gemäß einem der Ansprüche 1 bis 14, die in Wasser innerhalb eines Zeitraums von 2 min dispergierbar ist.

16. Tablette gemäß einem der vorhergehenden Ansprüche, worin die Menge an Lamotrigin 5 bis 250 mg beträgt.

17. Tablette gemäß einem der vorhergehenden Ansprüche, worin die Dispersion Teilchen mit einer Teilchengrößenverteilung von 100 % mit weniger als 710 µm und mehr als 50 % mit weniger als 300 µm enthält.

18. Tablette gemäß Anspruch 17, worin die Dispersion Teilchen mit einer Teilchengrößenverteilung von 100 % mit weniger als 710 µm, mehr als 70 % mit weniger als 300 µm und mehr als 50 % mit weniger als 200 µm enthält.

19. Tablette gemäß einem der vorhergehenden Ansprüche, worin die Tablette 25 bis 200 mg Lamotrigin oder ein Salz davon enthält und eine Formulierung umfaßt aus 30 % G/G bis 50 % G/G Lamotrigin oder einem Salz davon, 26 % G/G bis 46 % G/G Calciumcarbonat, 5 bis 30 % G/G Niederhydroxypropylcellulose-LH11 oder mikrokristalliner Cellulose, 0,25 % G/G bis 30 % G/G Magnesiumaluminiumsilicat oder Bentonit, 0,25 bis 5 % G/G Povidon oder 1 bis 8 % G/G vorgelierter Stärke, 0 bis 8 % G/G Natriumstärkeglykolat, 0,25 % G/G bis 2 % G/G Magnesiumstearat und optionalen Filmüberzugsmischungen aus 0,1 % G/G bis 2 % G/G Opadry und 0,1 % G/G bis 0,5 % G/G Polyethylenglykol.

20. Tablette gemäß Anspruch 19, worin die Formulierung 35 % G/G bis 45 % G/G Lamotrigin oder ein Salz davon, 31 % G/G bis 41 % G/G Calciumcarbonat, 5 bis 15 % G/G Niederhydroxypropylcellulose oder mikrokristalline Cellulose, 0,25 % G/G bis 10 % G/G Magnesiumaluminiumsilicat oder Bentonit, 0,5 bis 2 % G/G Povidon oder 2 bis 5 % G/G vorgelierte Stärke, 0 bis 5 % G/G Natriumstärkeglykolat, 0,25 % G/G bis 1 % G/G Magnesiumstearat und optionale Filmüberzugsmischungen aus 0,25 % G/G bis 1 % G/G Opadry und 0,1 % G/G bis 0,2 % G/G Polyethylenglykol beträgt.

21. Tablette gemäß einem der Ansprüche 1 bis 17, worin die Tablette 5 mg bis 50 mg Lamotrigin oder ein Salz davon enthält und die Formulierung 3 % G/G bis 13 % G/G Lamotrigin oder ein Salz davon, 50 % G/G bis 60 % G/G Lactose oder Calciumcarbonat, 20 % G/G bis 35 % G/G Niederhydroxypropylcellulose oder mikrokristalline Cellulose, 0 bis 8 % G/G Natriumstärkeglykolat, 0,25 % G/G bis 30 % G/G Magnesiumaluminiumsilicat oder Bentonit, 0,25 % G/G bis 5 % G/G Povidon oder 1 % G/G bis 8 % G/G vorgelierte Stärke, 0 bis 5 % G/G Natriumdocusat, 0 bis 3 % Natriumsaccharin, 0,25 % G/G bis 2 % G/G Magnesiumstearat und optionale Filmüberzugsmischungen aus 0,1 % G/G bis 2 % G/G Opadry und 0,1 % G/G bis 0,5 % G/G Polyethylenglykol umfaßt.

22. Tablette gemäß Anspruch 21, worin die Formulierung 5 % G/G bis 11 % G/G Lamotrigin oder ein Salz davon, 53 % G/G bis 59 % G/G Lactose oder Calciumcarbonat, 24 % G/G bis 30 % G/G Niederhydroxypropylcellulose oder mikrokristalline Cellulose, 0 bis 5 % G/G Natriumstärkeglykolat, 0,25 % G/G bis 10 % G/G Magnesiumaluminiumsilicat oder Bentonit, 0,5 % G/G bis 2 % G/G Povidon oder 2 % G/G bis 5 % G/G vorgelierte Stärke, 0,15 % G/G bis 0,5 % G/G Natriumdocusat, 0,5 % G/G bis 2 % Natriumsaccharin, 0,25 % G/G bis 1 % G/G Magnesiumstearat und optionale Filmüberzugsmischungen aus 0,25 % G/G bis 1 % G/G Opadry und 0,1 % G/G bis 0,2 % G/G Polyethylenglykol beträgt.

23. Verfahren zur Herstellung einer wasserdispergierbaren Tablette, umfassend 2 % bis 90 % G/G Lamotrigin oder ein pharmazeutisch akzeptables Salz davon und 0,25 bis 40 % G/G eines pharmazeutisch akzeptablen quellbaren Tons; wobei das Verfahren das In-Verbindung-Bringen von Lamotrigin mit dem quellbaren Ton zur Bildung von Granalien und das anschießende Verpressen der Granalien zur Bildung einer Tablette umfaßt, die in Wasser innerhalb eines Zeitraums von 3 min zur Bereitstellung einer Dispersion dispergierbar ist, die durch einen Siebfilter mit einer Maschenweite von 710 µm gemäß dem in der Britischen Pharmakopoe 1988, Band II, Seite 895 für dispergierbare Tabletten definierten Test gelangen kann.

24. Verfahren gemäß Anspruch 23, umfassend die Schritte aus:
a) Vermischen von Lamotrigin oder einem Salz davon und dem quellbaren Ton in trockener, feinverteilter Form, gegebenenfalls unter Zugabe eines oder mehrerer anderer pharmazeutischer Träger oder Zusatzstoffe;
b) Zugabe einer Menge von Granulierungsflüssigkeit, die ausreichend zum Anfeuchten der trockenen Mischung ist;
c) Granulierung der resultierenden feuchten Mischung zur Bildung von Granalien;
d) Trocknen der Granalien und gegebenenfalls Vermischen der Granalien mit anderen optionalen Trägern oder Zusatzstoffen wie Schmiermitteln, Gleitmitteln und Geschmacksstoffen; und
e) Verpressen der Granalien zur Bildung einer Tablette, die in Wasser innerhalb eines Zeitraums von 3 min zur Bereitstellung einer Dispersion dispergierbar ist, die durch einen Siebfilter mit einer Maschenweite von 710 µm gemäß dem oben definierten Test der Britischen Pharmakopoe für dispergierbare Tabletten gelangen wird.

25. Verfahren gemäß den Ansprüchen 23 oder 24, worin ein Bindemittel zum Lamotrigin oder einem Salz davon und dem quellbaren Ton hinzugegeben wird.

26. Verfahren gemäß einem der Ansprüche 23 bis 25, worin ein Sprengmittel zum Lamotrigin oder einem Salz davon oder dem quellbaren Ton hinzugegeben wird.

27. Verfahren gemäß einem der Ansprüche 23 bis 26, worin ein Füllstoff zu Lamotrigin oder einem Salz davon und dem quellbaren Ton hinzugegeben wird.

## Revendications

1. Comprimé dispersable dans l'eau comprenant 2% en poids/poids à 90% en poids/poids de lamotrigine ou d'un sel pharmaceutiquement acceptable de celle-ci et 0,25 à 40% en poids/poids d'une argile gonflable pharmaceutiquement acceptable qui est présente dans les granules du comprimé pour fonner un comprimé qui peut se disperser dans l'eau dans les limites d'une période de 3 minutes pour former une dispersion qui peut traverser un tamis avec une ouverture de maille de 710 µm suivant le test pour comprimés dispersables défini dans le British Pharmacopoeia, 1988, volume II, page 895.

2. Comprimé suivant la revendication 1, dans lequel l'argile gonflable est une smectite ou attapulgite.

3. Comprimé suivant la revendication 2, dans lequel la smectite est choisie dans le groupe des montmorillonoïdes.

4. Comprimé suivant la revendication 3, dans lequel le groupe des montmorillonoïdes est choisi dans le groupe comprenant la montmorillonite, la seuconite, la verniculite, la bentonite, l'hectorite et le silicate d'aluminium et de magnésium.

5. Comprimé suivant la revendication 4, dans lequel le montmorillonoïde est du silicate de magnésium-aluminium ou de la bentonite.

6. Comprimé suivant la revendication 5, dans lequel le silicate de magnésium-aluminium est du Veegum F.

7. Comprimé suivant l'une quelconque des revendications précédentes, dans lequel l'argile gonflable est présente dans les granules du comprimé en une quantité de 1 à 40% en poids/poids.

8. Comprimé suivant la revendication 7, dans lequel l'argile gonflable est présente en une quantité de 1 à 10% en poids/poids.

9. Comprimé suivant la revendication 8, dans lequel l'argile gonflable est présente en une quantité de 5 à 10% en poids/poids.

10. Comprimé suivant l'une quelconque des revendications précédentes, qui comprend de plus un agent de désagrégation.

11. Comprimé suivant l'une quelconque des revendications précédentes, dans lequel l'agent de désagrégation additionnel est du glycolate d'amidon de sodium ou une hydroxypropylcellulose inférieure.

12. Comprimé suivant l'une quelconque des revendications précédentes, qui comprend de plus un liant.

13. Comprimé suivant la revendication 12, dans lequel le liant est la povidone k30.

14. Comprimé suivant l'une quelconque des revendications précédentes, qui comprend de plus une charge.

15. Comprimé suivant l'une quelconque des revendications 1 à 14, qui peut se disperser dans l'eau dans les limites d'une période de 2 minutes.

16. Comprimé suivant l'une quelconque des revendications précédentes, dans lequel la quantité de lamotrigine est de 5 à 250 mg.

17. Comprimé suivant l'une quelconque des revendications précédentes, dans lequel la dispersion contient des particules ayant une distribution de tailles des particules de 100% inférieure à 710 µm, et de plus de 50% inférieure à 300 µm.

18. Comprimé suivant la revendication 17, dans lequel la dispersion contient des particules ayant une distribution de tailles des particules de 100% inférieure à 710 µm, de plus de 70% inférieure à 300 µm, et de plus de 50% inférieure à 200 µm.

19. Comprimé suivant l'une quelconque des revendications précédentes, dans lequel le comprimé contient 25 à 200 mg de lamotrigine ou d'un sel de celle-ci et comprend une formulation de 30% en poids/poids à 50% en poids/poids de lamotrigine ou d'un sel de celle-ci, 26% en poids/poids à 46% en poids/poids de carbonate de calcium, 5 à 30% en poids/poids d'hydroxypropylcellulose inférieure LH11 ou de cellulose microcristalline, 0,25% en poids/poids à 30% en poids/poids de silicate de magnésium-aluminium ou de bentonite, 0,25 à 5% en poids/poids de povidone ou 1 à 8% en poids/poids d'amidon prégélifié, 0 à 8% en poids/poids de glycolate d'amidon de sodium, 0,25% en poids/poids à 2% en poids/poids de stéarate de magnésium et d'éventuels composites d'enrobage formant film à raison de 0,1% en poids/poids à 2% en poids/poids d'Opadry et de 0,1% en poids/poids à 0,5% en poids/poids de polyéthylène glycol.

20. Comprimé suivant la revendication 19, dans lequel la formulation est de 35% en poids/poids à 45% en poids/poids de lamotrigine ou d'un sel de celle-ci, de 31% en poids/poids à 41% en poids/poids de carbonate de calcium, de 5 à 15% en poids/poids d'hydroxypropylcellulose inférieure ou de cellulose microcristalline, de 0,25% en poids/poids à 10% en poids/poids de silicate de magnésium-aluminium ou de bentonite, de 0,5 à 2% en poids/poids de povidone ou de 2 à 5% en poids/poids d'amidon prégélifié, de 0 à 5% en poids/poids de glycolate d'amidon de sodium, de 0,25% en poids/poids à 1% en poids/poids de stéarate de magnésium et d'éventuels composites d'enrobage formant film à raison de 0,25% en poids/poids à 1% en poids/poids d'Opadry et de 0,1% en poids/poids à 0,2% en poids/poids de polyéthyléne glycol.

21. Comprimé suivant l'une quelconque des revendications 1 à 17, dans lequel le comprimé contient 5 mg à 50 mg de lamotrigine ou d'un sel de celle-ci et la formulation comprend 3% en poids/poids à 13% en poids/poids de lamotrigine ou d'un sel de celle-ci. 50% en poids/poids à 60% en poids/poids de lactose ou de carbonate de calcium, 20% en poids/poids à 35% en poids/poids d'hydroxypropylcellulose inférieure ou de cellulose microcristalline, 0 à 8% en poids/poids de glycolate d'amidon de sodium, 0,25% en poids/poids à 30% en poids/poids de silicate de magnésium-aluminium ou de bentonite, 0,25% en poids/poids à 5% en poids/poids de povidone ou 1% en poids/poids à 8% en poids/poids d'amidon prégélifié, 0 à 5% en poids/poids de docusate de sodium, 0 à 3% en poids/poids de saccharine sodium, 0,25% en poids/poids à 2% en poids/poids de stéarate de magnésium et d'éventuels composites d'enrobage formant film à raison de 0,1% en poids/poids à 2% en poids/poids d'Opadry et de 0,1% en poids/poids à 0,5% en poids/poids de polyéthylène glycol.

22. Comprimé suivant la revendication 21, dans lequel la formulation est de 5% en poids/poids à 11% en poids/poids de lamotrigine ou d'un sel de celle-ci, de 53% en poids/poids à 59% en poids/poids de lactose ou de carbonate de calcium, de 24% en poids/poids à 30% en poids/poids d'hydroxypropylcellulose inférieure ou de cellulose micro-cristalline, de 0 à 5% en poids/poids de glycolate d'amidon de sodium, de 0,25% en poids/poids à 10% en poids/poids de silicate de magnésium-aluminium ou de bentonite, de 0,5% en poids/poids à 2% en poids/poids de povidone ou de 2% en poids/poids à 5% en poids/poids d'amidon prégélifié, de 0,15% en poids/poids à 0,5% en poids/poids de docusate de sodium, de 0,5% en poids/poids à 2% en poids/poids de saccharine sodium, de 0,25% en poids/poids à 1% en poids/poids de stéarate de magnésium et d'éventuels composites d'enrobage formant film à raison de 0,25% en poids/poids à 1% en poids/poids d'Opadry et de 0,1% en poids/poids à 0,2% en poids/poids de polyéthylène glycol.

23. Procédé de préparation d'un comprimé dispersable dans l'eau comprenant 2% à 90% en poids/poids de lamotrigine ou d'un sel pharmaceutiquement acceptable de celle-ci et 0,25 à 40% en poids/poids d'une argile gonflable pharmaceutiquement acceptable, ledit procédé consistant à amener de la lamotrigine en association avec ladite argile gonflable pour former des granules et ensuite à comprimer les granules pour former un comprimé qui peut se disperser dans l'eau dans les limites d'une période de 3 minutes pour former une dispersion qui peut traverser un tamis avec une ouverture de maille de 710 µm suivant le test pour comprimés dispersables défini dans le British Pharmacopoeia, 1988, volume II, page 895.

24. Procédé suivant la revendication 23, comprenant les étapes suivantes :
a) le mélange sous une forme finement divisée, sèche de lamotrigine ou d'un sel de celle-ci et de l'argile gonflable, éventuellement avec l'addition d'un ou plusieurs autres supports ou excipients pharmaceutiques;
b) l'addition d'une quantité de fluide de granulation suffisante pour humidifier le mélange sec;
c) la granulation du mélange humide résultant pour former des granules;
d) le séchage des granules et éventuellement le mélange des granules avec d'autres supports ou excipients éventuels tels que des lubrifiants, des agents de glissement et des agents aromatisants; et
e) la compression des granules pour former un comprimé qui peut se disperser dans l'eau dans les limites d'une période de 3 minutes pour former une dispersion qui traversera un tamis avec une ouverture de maille de 710 µm suivant le test du British Pharmacopoeia défini ci-dessus pour comprimés dispersables.

25. Procédé suivant l'une ou l'autre des revendications 23 et 24, dans lequel un liant est ajouté à la lamotrigine ou à un sel de celle-ci et à l'argile gonflable.

26. Procédé suivant l'une quelconque des revendications 23 à 25, dans lequel un agent de désagrégation est ajouté à la lamotrigine ou à un sel de celle-ci et à l'argile gonflable.

27. Procédé suivant l'une quelconque des revendications 23 à 26, dans lequel une charge est ajoutée à la lamotrigine ou à un sel de celle-ci et à l'argile gonflable.
